# EUROPÄISCHE PATENTSCHRIFT

(11) **EP 1 658 819 B1**
(45) Veröffentlichungstag und Bekanntmachung des Hinweises auf die Patenterteilung: **02.01.2008**
(21) Anmeldenummer: 04027430.0
(22) Anmeldetag: 18.11.2004
(51) Int. Cl.: A61B 19/00

(54) **Transparente Markerumhüllung**
Transparent marker cover
Enveloppe transparente pour un marqueur

(43) Veröffentlichungstag der Anmeldung: 24.05.2006
(73) Patentinhaber: BrainLAB AG, 85551 Kirchheim/Heimstetten (DE)
(72) Erfinder: Feilkas, Thomas, 85567 Grafing (DE); Huber, Hansjörg, 80805 München (DE)
(74) Vertreter: Schwabe - Sandmair - Marx

(56) Entgegenhaltungen:
- EP-A- 0 566 861
- WO-A-99/04719
- WO-A-99/40869
- US-A- 5 524 643
- US-A- 5 588 430
- US-A- 5 816 252

## Beschreibung

Die vorliegende Erfindung betrifft eine transparente Umhüllung für Marker, die für Trackingsysteme, insbesondere für medizinische Trackingsysteme (auch Navigationssysteme genannt) verwendet werden. Die Trackingsysteme dienen dazu, Positionen von Vorrichtungen zu bestimmen und zu verfolgen, indem die Position von Markern, die an der Vorrichtung angebracht sind, detektiert werden (z.B. mittels Kamera). Die medizinischen Trackingsysteme dienen insbesondere dazu, die Position eines Instruments oder eines Körperteils mit Hilfe von Markern und/oder eines daran angebrachten Referenzsterns zu bestimmen. Dieser Referenzstern weist die Marker auf. Die Marker sind entweder passive oder aktive Marker. Passive Marker reflektieren Licht, insbesondere Infrarotlicht (z.B. aber auch sichtbares oder UV-Licht), das auf sie gestrahlt wird. Aktive Marker senden von selbst Licht aus. Die Position der Marker, insbesondere des Referenzsterns und damit des Instruments oder Körperteils wird mit Hilfe von Kameras bestimmt, die das von den Markern ausgesandte Licht detektieren.

Bei den Markern handelt es sich typischerweise um kugelförmige Marker, sie können aber auch eine andere Gestalt, wie z.B. würfelförmig, aufweisen. Insbesondere passive Marker sind an ihrer Oberfläche katzenaugenähnlich mit einer Vielzahl von kleinen Reflexionsflächen ausgebildet, die selbst wiederum kugelförmig oder halbkugelförmig sein können. Dadurch befinden sich eine Vielzahl von Vertiefungen auf den Markern, in denen sich Flüssigkeit , insbesondere Spülflüssigkeit, Blut oder Blutverschmierungen anlagern können. Dies kann die Abstrahlung von Licht von den Markern verändern. Dadurch kann das Navigationssystem die tatsächliche Position der Marker falsch berechnen. Insbesondere kann bei beispielsweise kugelförmigen Markern das Zentrum der Markerkugel falsch berechnet werden, falls das abgestrahlte Licht (aktives Licht oder reflektiertes Licht) durch die auf den Markern befindlichen Flecken oder Flüssigkeitstropfen geändert wird. Insbesondere kann es an der Oberfläche der Flüssigkeit zu zusätzlichen Reflexionen kommen, die die Abstrahlung beeinträchtigen.

Problematisch ist bei der praktischen medizinischen Anwendung insbesondere, dass auf Grund der Vielzahl von Vertiefungen es schwer für den Praktiker (insbesondere Arzt oder Krankenschwester) erkennbar ist, ob sich Flüssigkeit oder Flecken auf dem Marker befinden. Somit weiß der Praktiker nicht, ob eine Reinigung der Marker notwendig ist. Insbesondere sorgen die Vertiefungen dafür, dass sich die Flüssigkeit auf der Oberfläche des Markers ausbreitet und schlechter zu sehen ist.

Aus US 5,588,430 ist ein rahmenloses stereotaktisches Verfahren bekannt das auf dem Gebiet der Zahnmedizin verwendet wird. Dabei werden LEDs als Marker verwendet. Eine Markerplatte kann aus transparentem Plastik hergestellt sein und darin angeordnet die LEDs aufweisen oder die LEDs können auf einer Oberfläche der Markerplatte montiert sein.

Aus WO 99/40869 ist eine Nadel-Lokalisienmosanordnun bekannt um eine Knochen schädigung zu markieren. In der Lokalisierungsnadel befindet sich eine radioaktive Strahlungsquelle die mittels Nuklearmedizin erkannt werden kann Das hierzu verwendete flüssitze radioaktive Strahlungsmittel befindet sich in einer mittels Schraubengewinden verschlossenen und abgedichteten Kammer.

Aufgabe der Erfindung ist es, eine Vorrichtung, ein System mit Markern und ein Trackingsystem so zu verwenden, dass es leichter ist, eine Störung der optischen Abstrahlungseigenschaften der Marker zu erkennen und/oder bei dem zu beseitigen und/oder eine derartige Beeinträchtigung unwahrscheinlicher ist.

Vorstehende Aufgabe wird durch den Gegenstand des unabhängigen Anspruchs 1 gelöst. Die abhängigen Ansprüche sind auf vorteilhafte Ausführungsformen gerichtet.

Erfindungsgemäß wird eine transparente Markerumhüllung verwendet. Diese erlaubt es, die vorgenannten Probleme zu lösen. Vorzugsweise sind zumindest Abschnitte der Markerumhüllung, insbesondere die gesamte Markerumhüllung aus einem transparenten Material ausgebildet. Dieses transparente Material ist für diejenige (insbesondere optische) Strahlung transparent, die von Kameras des Navigationssystems detektiert wird, um die Position der Marker zu bestimmen. Die Strahlung geht von den Markern aus, weil sie von diesen reflektiert wird (passive Marken) oder von diesem abgestrahlt wird (aktive Marken). Insbesondere ist das Material für Infrarotstrahlung und/oder sichtbares Licht transparent.

Transparent bedeutet hierbei insbesondere, dass ein Transmissionsgrad von 80% oder mehr, bevorzugt 90% oder mehr, besonders bevorzugt 95% oder mehr und höchst bevorzugt 99% oder mehr, für die zu detektierende Strahlung besteht. Insbesondere bedeutet transparent, dass der Reflexionsgrad für die zu detektierende Strahlung bevorzugt 20% oder weniger, besonders bevorzugt 10% oder weniger, noch mehr bevorzugt 5% oder weniger und höchst bevorzugt 1% oder weniger, beträgt. Insbesondere bedeutet transparent, dass der Absorptionsgrad für die zu detektierende Strahlung 20% oder weniger, bevorzugt 10% oder weniger, besonders bevorzugt 5% oder weniger und höchst bevorzugt 1% oder weniger beträgt.

Bevorzugt weist die Markerumhüllung eine Oberfläche auf, die Flüssigkeit nicht aufnimmt, insbesondere nicht aufsaugt, insbesondere nicht durch Oberflächenspannungseffekte anzieht oder hält. Insbesondere ist die Markerumhüllung so ausgebildet, dass sie eine glatte, insbesondere zumindest abschnittsweise ebene Oberfläche aufweist. Bevorzugt ist die Oberfläche flüssigkeitsabweisend, insbesondere hydrophob ausgebildet. Insbesondere ist die Oberfläche und/oder das Material der Markerumhüllung aus einem Polymer, aus Polyvinylchlorid, aus Polyethylen, aus Polypropylen, aus Polycarbonat, oder aus Tetrafluorethylen oder einer Mischung aus dem vorgenannten ausgebildet. Die Oberfläche kann insbesondere silanisiert sein. Die Markerumhüllung kann auch so ausgebildet sein, dass sie im Infraroten transparent ist, aber im Sichtbaren nicht transparent ist. Insbesondere kann sie im Sichtbaren reflektierend sein und/oder weiß erscheinen, um so Verschmutzungen für den Praktiker leichter erkennen zu können, während vom Marker ausgestrahlte und detektierte Strahlen im Infraroten durch die Markerumhüllung hindurch gelangen.

Vorzugsweise ist die Markerumhüllung in der Art eines Gehäuses ausgebildet, das einen Marker oder die Markeranordnung (Referenzstern) zumindest teilweise und vorzugsweise vollständig umgibt.

Die Markerumhüllung kann auch in der Art einer Schutzschicht, die die Oberfläche des Markers glättet, den Marker teilweise umhüllen. Die Schutzschicht ist vorzugsweise direkt auf der Markeroberfläche aufgebracht.

Die Markerumhüllung hat vorzugsweise eine stabile insbesondere starre Oberflächengestalt, um Verschmutzungen der Oberfläche leichter erkennen zu können und dies leichter abwischen zu können. Die Markerumhüllung nimmt vorzugsweise eine vorgeformte Gestalt ein. Sie ist insbesondere aus einem dünnen Material, insbesondere Kunststoffmaterial, ausgebildet. Dabei bedeutet "dünn" vorzugsweise weniger als 2 mm, insbesondere weniger als 1 mm, vorzugsweise weniger als 0,5 mm. Die Markerumhüllung kann Verstärkungen, Falze oder Falten aufweisen, um die Gestalt zu stabilisieren. Diese Verstärkungen, Falten oder Falze sind vorzugsweise so angeordnet, dass sie eine Detektion der Marker nicht stören.

Vorzugsweise ist die Markerumhüllung mit einer vorgeformten Gestalt ausgebildet, die eine oder mehrere Vertiefungen aufweist, um einen oder mehrere Marker oder einen Referenzstern mit Marker oder z. B. eine Kombination aus zwei Markern (z. B. für einen Pointer) aufzunehmen. Ein Referenzstern umfasst insbesondere mindestens 2 oder 3 Marker, die durch mechanische Verbindungsglieder so verbunden sind, dass die Marker insbesondere eine Ebene (3 Marker) oder eine 3D-Gestalt (4 oder mehr Marker) aufspannen. Insbesondere ist die Vertiefung in der Gestalt so ausgebildet, dass die Marker bzw. der Referenzstern eingebettet wird. Dies bedeutet insbesondere, dass nach Einbringen des mindestens einen Markers oder des Referenzsterns in die Vertiefung der Markerumhüllung ein Deckel, insbesondere flacher Deckel die Vertiefung verschließen kann, um so den mindestens einen Marker durch Deckel und Vertiefung vollständig zu umschließen.

Vorzugsweise ist die Markerumhüllung so gestaltet, dass mindestens ein Marker und/oder ein Referenzstern in die Markerumhüllung einbringbar ist, insbesondere auch wieder entnehmbar ist. Vorzugsweise weist die Markerumhüllung den vorgenannten Verschluss und/oder einen Deckel auf, der insbesondere ebenfalls transparent ist und vorzugsweise aus demselben Material ausgebildet ist. Der Verschluss ist vorzugsweise so gestaltet, dass der sich in der Ausnehmung befindliche mindestens eine Marker und/oder Referenzstern aus der Ausnehmung nur entnommen werden kann, wenn der Verschluss geöffnet wird. Vorzugsweise ist die Umhüllung so gestaltet, dass die Öffnung, die die Ausnehmung bildet, durch den Verschluss verschlossen wird. Der Verschluss ist besonders vorzugsweise so gestaltet, dass die Umhüllung abdichtend verschlossen wird, so dass keine Flüssigkeiten, insbesondere verschmutzende Flüssigkeiten in die Umhüllung eintreten können.

Vorzugsweise weist die Markerumhüllung ein Mittel auf, um den mindestens einen Marker und/oder Referenzstern an einer vorgegebenen Position zu halten. Dazu kann eine Klemm- oder Presspassung und/oder ein Einschnappmechanismus vorgesehen werden, der den mindestens einen Marker und/oder Referenzstern hält, wenn dieser in die Markerumhüllung, insbesondere in die Ausnehmung eingebracht wird. Insbesondere kann auch der zuvor erwähnte Verschluss dazu ausgebildet sein, den mindestens einen Marker und/oder Referenzstern an der vorgesehenen Position zu halten.

Vorzugsweise umfasst die Markerumhüllung einen Sensor, um Fremdstoffe zu erkennen oder nachzuweisen, die von den Markern ausgehende Strahlen beeinträchtigen können. Insbesondere ist der Sensor vorzugsweise so ausgebildet, dass er Flüssigkeiten oder Fremdstoffe an der Oberfläche der Markerumhüllung, insbesondere der Außenoberfläche der Markerumhüllung nachweisen oder detektieren kann. Der Sensor ist vorzugsweise so ausgebildet, dass er bei Nachweis eines Fremdstoffes oder einer Flüssigkeit ein detektierbares Signal, insbesondere ein optisches oder elektrisches Signal abgibt. Auf diese Art und Weise kann das Navigationssystem gewarnt werden, dass die von den Markern abgestrahlten Strahlen möglicherweise gestört oder beeinträchtigt sind. Auch kann der Sensor oder das Nachweismittel für Feuchtigkeit oder Fremdstoffe so ausgebildet sein, dass es sich für die Beobachtungskameras feststellbar optisch verändert. Dadurch wird eine zusätzliche Warnelektronik vermeidbar. Insbesondere kann die Oberfläche der Markerumhüllung so gestaltet sein, dass es sich bei Anfeuchtung verfärbt, so dass beispielsweise die Markerumhüllung schwarz und/oder für das zu detektierende Licht (z.B. IR-Licht) nicht mehr transparent wird, wenn sie mit Flüssigkeit in Kontakt kommt. Somit ist sichergestellt, dass keine fehlerhafte Navigation durchgeführt wird, da der Benutzer oder Praktiker sofort erkennt, dass die Abstrahlung von den Markern möglicherweise gestört ist. Er kann die Markerumhüllung wieder reinigen, um so die Störung zu beseitigen.

Die transparente Markerumhüllung kann getrennt von den Markern oder Referenzsternen bereitgestellt werden. Die Marker oder Referenzsterne können aber auch in Kombination mit der Markerumhüllung angeboten werden. Insbesondere kann ein System aus Marker und Markerumhüllung auch so ausgebildet sein, dass die transparente Markerumhüllung fest um den mindestens einen Marker oder Referenzstern herum ausgebildet ist, insbesondere verschweißt ist oder der mindestens eine Marker und/oder Referenzstern in eine transparente Markerumhüllung eingegossen ist. Beispielsweise kann der mindestens eine Marker und/oder Referenzstern in eine transparente Kunststoffmasse eingegossen sein.

Vorzugsweise wird ein Trackingsystem (auch Navigationssystem genannt) bereitgestellt, das einen Referenzstern und Kameras zur Detektion optischer Strahlung von den Markern des Referenzsterns umfasst, wobei mindestens einer der Marker und/oder der Referenzstern durch die erfindungsgemäße transparente Markerumhüllung umgeben sind. Im Falle passiver Marker ist vorzugsweise eine Marker-Beleuchtung vorgesehen, die Licht, insbesondere IR-Licht auf die Marker abstrahlt, das von diesen reflektiert wird. Vorzugsweise ist das Trackingsystem zum Navigieren eines medizinischen Instruments oder zum Feststellen einer Position eines Körperteils so ausgebildet, dass ein Warnsignal abgegeben wird, wenn ein Sensor Fremdstoffe oder Flüssigkeiten feststelle, die die Detektion der optischen Strahlung von den Markern beeinträchtigt, wie oben ausgeführt wurde. Das Trackingsystem kann aber auch in anderen Gebieten, wie z. B. Virtual Reality (z. B. Computer- oder Viedeospiele), Robotersteuerung, Sportmedizin usw. Anwendung finden.

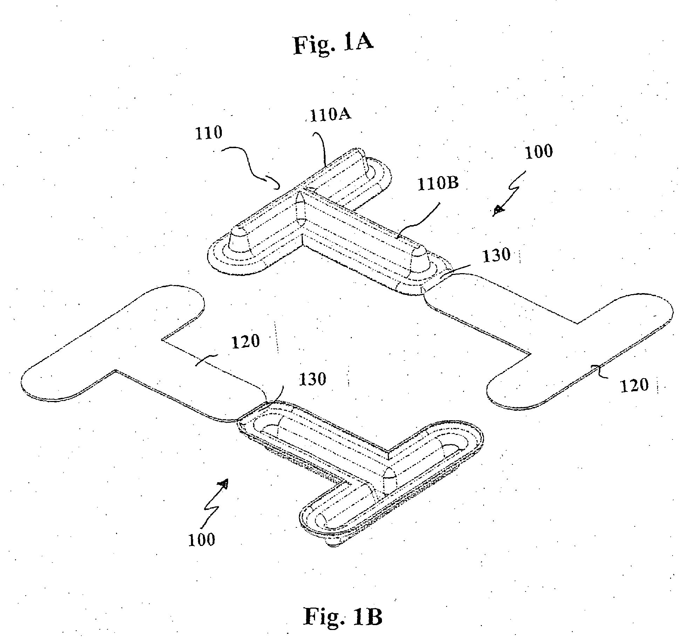

Im folgenden wird eine Ausführungsform der vorliegenden Erfindung erläutert. Dabei werden weitere Merkmale der Erfindung offenbart. Fig. 1A und 1B zeigen eine transparente Markerumhüllung gemäß einer Ausführungsform;
- Fig. 2: zeigt eine negative Tiefziehform für eine transparente Markerumhüllung gemäß einer weiteren Ausführungsform;
- Fig. 3: zeigt eine Draufsicht auf die Ausführungsform der Fig. 1;
- Fig. 4: zeigt eine Untenansicht auf die Ausführungsform der Fig. 1;
- Fig. 5: zeigt eine Seitenansicht der Ausführungsform der Fig. 1.

Fig. 1A und 1B zeigen eine transparente Markerumhüllung 100 . Die Markerumhüllung 100 weist eine T- förmige Gestalt 110, die durch zwei schalenförmige Längsgräben 110A und 110B gebildet wird. Die einander übergehen. Die Form der Gräben wird zum Rand hin flacher. Mit einem Längsgraben 110B ist an dem Ende, das dem Längsgraben 110A abgewandt ist ein Deckel 120 durch ein Schanier 130 verbunden. Der Deckel ist vorzugsweise flach und/oder eben ausgebildet und verschließt die durch die beiden Längsgräben 110A und 110B ausgebildete T-förmige Öffnung. Die Längsgräben 110A und 110B stellen eine Vertiefung, insbesondere schalenartige Vertiefung dar. Die Markerumhüllung, insbesondere sämtliche Bestandteile wie Deckel 120, Längsgräben 110 und Scharnier 130 ist vorzugsweise aus Polypropylen ausgebildet, wie es beispielsweise für pharmazeutische Verpackungen verwendet wird. Die Foliendicke ist vorzugsweise kleiner als 1 mm, insbesondere kleiner als 500 µm, beispielsweise 300 µm. Die Markerumhüllung wird vorzugsweise aus einer Folie gebildet und mittels eines Tiefziehverfahrens gebildet. Vorzugsweise entsprechen die Markerumhüllung und die dort eingesetzten Rohstoffe den einschlägigen BgVV-, EG- und FDA-Bestimmungen für die Herstellung von Pharmaverpackungen. Vorzugsweise ist die physiologische Unbedenklichkeit der Folie gewährleistet. Vorzugsweise ist das Folienmaterial so gestaltet, dass der Kristallschmelzbereich nach ISO 3146 bei über 100°C liegt. Vorzugsweise ist die mechanische Reißfestigkeit nach ISO 527-3 über 10 N/mm². Vorzugsweise ist der Oberflächenglanz nach DIN 67530 >70%, besonders vorzugsweise >90%. Vorzugsweise ist die Trübung (Haze) nach ASTM D-1003 <30%, besonders vorzugsweise <20%.

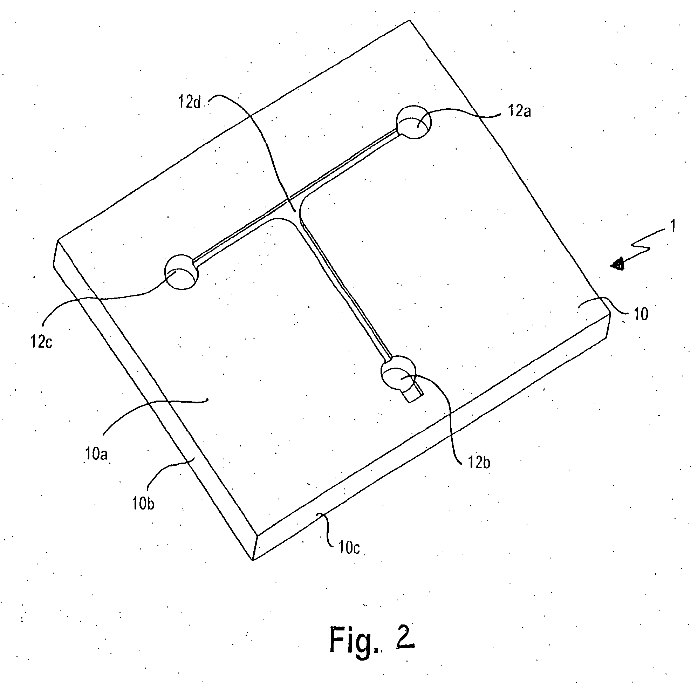

In der Tiefziehform gemäß Fig. 2 für eine transparente Markerumhüllung ist vorzugsweise eine Ausnehmung ausgebildet, die Ausnehmungen 12a, 12b und 12c für Markerkugeln umfasst. Die Ausnehmungen sind vorzugsweise zylindrisch ausgebildet, um entsprechende zylinderähnliche Vertiefung in der Markerumhüllung auszubilden, um ein einfaches Einführen der Markerkugeln zu ermöglichen. Vorzugsweise umfasst die Ausnehmung 12 einen Abschnitt 12d, der bei der Formung der Markerumhüllung durch die Tiefziehform eine kanalförmige Vertiefung zu bilden, die für das Einbringen der Verbindungsarme des Referenzsterns zu den einzelnen Markerkugeln vorgesehen ist. Bei der in Fig. 1 gezeigten Ausführungsform kann somit bei einer damit hergestellten Markerumhüllung ein gesamter Referenzstern in die der Ausnehmung 12 entsprechende Vertiefung eingebracht werden. Die so hergestellte Markerumhüllung kann ebenso wie die Ausführungsform gemäß Fig. 1 einen Referenzstern mit einer so genannten T-Geometrie aufnehmen.

Die Marker müssen nicht kugelförmig sein, sondern können z. B. auch flach, z. B. plättchen-ähnlich oder wie eine Scheibe ausgebildet sein. Die flachen Marker weisen vorzugsweise eine rauhe Oberfläche mit einer Vielzahl von reflektierenden Erhebungen auf.

Anwendungen der Erfindung liegen insbesondere bei Kniegelenkkreuzband-Operationen (ACL-Operationen), bei Tracking- oder Navigationsanwendungen wie den so genannten Vector Vision Spine, also einer Navigationsanwendung auf dem Gebiet der Wirbelsäule und dem so genannten Vector Vision Trauma (VV-Trauma) und der Radiotherapie. Andere medizinische und nicht-medizinische Anwendungen betreffen jegliche Art der Bewegungsanalyse und Bewegungserfassung von Körpern und Vorrichtungen. Beispiele sind Viedeospiele, Fernseh- und Filmanimationen, Virtual Reality, Ganganalyse (insbesondere beim Menschen oder Tier), Körperbewegungsanalyse (Sportmedizin), Analyse von Prothesen, Neurowissenschaften, Produkt-Design, usw.

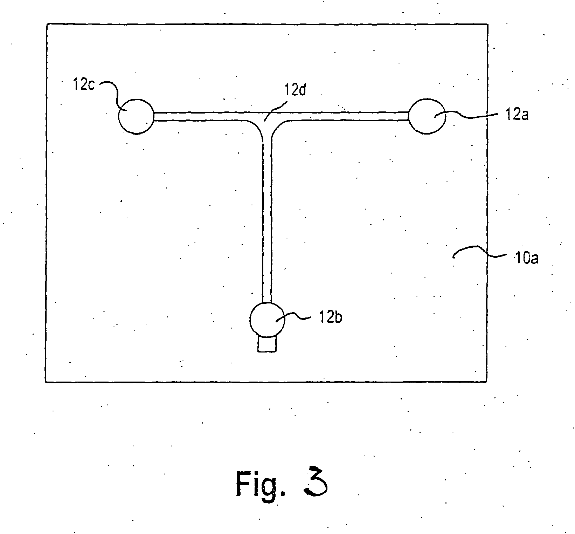

Fig. 3 zeigt eine Draufsicht der Tiefziehform der Fig. 2 Gleiche Teile sind mit gleichen Bezugszeichen versehen.

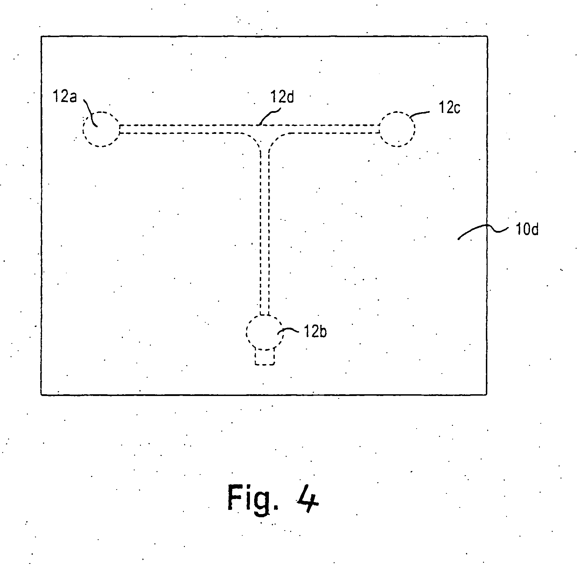

Fig. 4 zeigt eine Ansicht der Tiefziehform von unten, wobei gleiche Teile mit gleichen Bezugszeichen versehen sind. Die Unterseite des Quaders wurde mit dem Bezugszeichen 10d bezeichnet. Die Ausnehmung 12 mit den Bestandteilen 12a, 12b, 12c und 12d ist gestrichelt gezeichnet, da wobei sie sich im Gegensatz zu Fig. 2 und Fig. 3 nicht zur Oberfläche 10d hin öffnet. In Fig. 2 und Fig. 3 ist die Ausnehmung 12 durchgehend gezeichnet, da die Oberfläche 10a durch die Ausnehmung 12 unterbrochen ist.

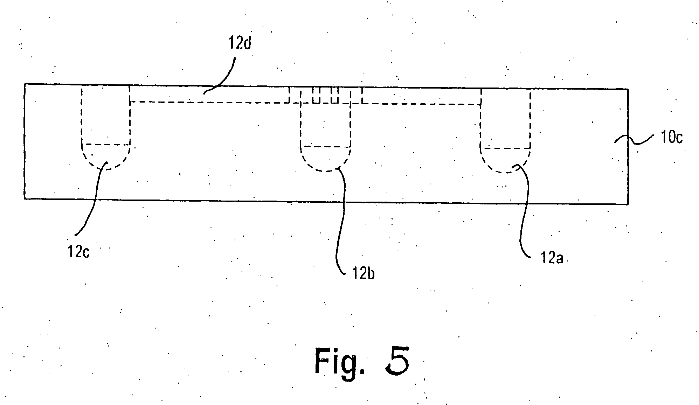

Fig. 5 zeigt eine Seitenansicht der Tiefziehform der Fig. 2 . Gleiche Teile sind wiederum mit gleichen Bezugszeichen versehen. Die Ausnehmung 12 mit den Bestandteilen 12a, 12b, 12c und 12d ist wiederum strichliert, da sie sich nicht zur Seitenfläche 10c öffnen Die in Figur 2 bis 5 gezeigte Tiefziehform ist eine Negativform.

## Patentansprüche

1. Verwendung einer transparenten Markerumhüllung (100) für medizinische Zwecke auf dem Gebiet der Bewegungsanalyse und/oder Bewegungsverfolgung von Geräten und/oder Körpern und/oder Teilen davon, wobei die Markerumhüllung ein Material aufweist, das für von mindestens einem Marker ausgehende optische Strahlen transparent ist, und wobei die Markerumhüllung so ausgebildet ist, dass sie den mindestens einen Marker mindestens teilweise umgibt, **dadurch gekennzeichnet, dass** die Umhüllung verschließbar ist und durch Öffnen des Verschlusses (110) der mindestens eine Marker und/oder der Referenzstern einbringbar oder entnehmbar ist und die Umhüllung (100) einen Feuchtigkeitssensor aufweist, der Feuchtigkeit oder Flüssigkeit auf der Außenseite der Umhüllung feststellen kann und darauf basierend ein Signal abgibt oder sich für Beobachtungskameras bei Kontakt mit Feuchtigkeit oder Flüssigkeit feststellbar optisch verändert.

2. Verwendung nach Anspruch 1, wobei das Material eine Flüssigkeit nicht aufnehmende, insbesondere glatte und/oder flüssigkeitsabweisende Oberfläche aufweist.

3. Verwendung nach einem der vorhergehenden Ansprüche, wobei die Markerumhüllung eine vorgeformte, stabile Gestalt (110, 120) aus insbesondere dünnem Kunststoffmaterial aufweist.

4. Verwendung nach einem der vorhergehenden Ansprüche, wobei die Markerumhüllung ausgebildet ist, einen Referenzstern mit Markern zumindest teilweise zu umgeben.

5. Verwendung nach einem der vorhergehenden Ansprüche, wobei die Umhüllung eine Vertiefung (110A, 110B) aufweist, in der mindestens ein Marker aufgenommen werden kann, um ihn mit der Umhüllung zumindest teilweise zu umgeben.

6. Verwendung nach einem der vorhergehenden Ansprüche, wobei die Umhüllung (100) so ausgebildet ist, dass, wenn der mindestens eine Marker an einer für ihn vorgesehenen Position innerhalb der Umhüllung (100) ist, der mindestens eine Marker verlagerungsfest relativ zur Umhüllung ist.

7. Verwendung nach einem der vorhergehenden Ansprüche, wobei mindestens ein Marker für medizinische Navigationszwecke verwendet wird.

8. Verwendung nach einem der vorhergehenden Ansprüche, wobei die von der Markerumhüllung umgebenen Marker für ein Trackingsystem und Kameras zur Detektion von optischer Strahlung von den umhüllten Markern verwendet werden.

9. Verwendung nach Anspruch 8, wobei das Trackingsystem ein Warnsignal abgibt und/oder der mindestens einer der umhüllten Marker nicht mehr detektierbar ist, wenn der Sensor Feuchtigkeit oder Flüssigkeit feststellt oder damit in Kontakt kommt.

10. Verwendung nach einem der vorhergehenden Ansprüche, bei welchem die optischen Strahlen auf Grund Reflexion oder Abstrahlung von dem mindestens einen Marker ausgeht.

## Claims

1. The use of a transparent marker casing (100) for medical purposes in the area of analysing and/or tracking the movement of apparatus and/or bodies and/or parts of the same, wherein the marker casing comprises a material which is transparent to optical radiation emanating from at least one marker, and wherein the marker casing is formed such that it at least partially surrounds the at least one marker, **characterised in that** the casing can be sealed and the at least one marker and/or the reference star can be inserted or taken out by opening the seal (110), and the casing (100) comprises a moisture sensor which can detect moisture or liquid on the outer side of the casing and outputs a signal on this basis or is optically altered in a way which observation cameras can detect, in the event of contact with moisture or liquid.

2. The use according to claim 1, wherein the material exhibits a surface which does not accommodate liquid, in particular a smooth and/or liquid-resistant surface.

3. The use according to any one of the preceding claims, wherein the marker casing comprises a preformed, stable structure (110, 120) made in particular of thin plastic material.

4. The use according to any one of the preceding claims, wherein the marker casing is formed to at least partially surround a reference star comprising markers.

5. The use according to any one of the preceding claims, wherein the casing comprises a recess (110A, 110B) in which at least one marker can be accommodated, in order to at least partially surround it with the casing.

6. The use according to any one of the preceding claims, wherein the casing (100) is formed such that, when the at least one marker is at a position provided for it within the casing (100), the at least one marker is fixed against shifting relative to the casing.

7. The use according to any one of the preceding claims, wherein at least one marker is used for medical navigation purposes.

8. The use according to any one of the preceding claims, wherein the markers surrounded by the marker casing are used for a tracking system and cameras for detecting optical radiation from the enveloped markers.

9. The use according to claim 8, wherein the tracking system outputs a warning signal and/or the at least one enveloped marker is no longer detectable when the sensor detects or comes into contact with moisture or liquid.

10. The use according to any one of the preceding claims, wherein the optical radiation emanates from the at least one marker because it is reflected by the at least one marker or because is emitted by at least one marker.

## Revendications

1. Utilisation d'une enveloppe de repère (100) transparente à des fins médicales dans le domaine de l'analyse du mouvement et/ou poursuite du mouvement d'appareils et/ou de corps et/ou de parties de ceux-ci, l'enveloppe de repère présentant un matériau qui est transparent pour des rayons optiques partant d'au moins un repère et l'enveloppe de repère étant réalisée de manière à entourer au moins en partie le au moins un repère, **caractérisée en ce que** l'enveloppe peut être fermée et le au moins un repère et/ou l'étoile de référence peut être introduite ou retirée par ouverture de la fermeture (110) et l'enveloppe (100) comporte une sonde d'humidité qui permet de constater de l'humidité ou un liquide sur la face extérieure de l'enveloppe, et délivre un signal sur la base de ceci ou change optiquement, d'une manière qui peut être constatée par des caméras d'observation, en cas de contact avec l'humidité ou le liquide.

2. Utilisation selon la revendication 1, dans laquelle le matériau présente une surface qui n'absorbe pas les liquides, en particulier une surface lisse et/ou hydrofuge.

3. Utilisation selon l'une des revendications précédentes dans laquelle l'enveloppe de repère présente une forme (110, 120) stable, préformée en une matière plastique en particulier mince.

4. Utilisation selon l'une des revendications précédentes, dans laquelle l'enveloppe de repère est conçue pour entourer au moins en partie une étoile de référence avec des repères.

5. Utilisation selon l'une des revendications précédentes, dans laquelle l'enveloppe présente un creux (110A, 110B) dans lequel peut-être reçu au moins un repère, afin de l'entourer au moins en partie avec l'enveloppe.

6. Utilisation selon l'une des revendications précédentes, dans laquelle l'enveloppe (100) est conçue de manière que le au moins un repère soit immobilisé par rapport à l'enveloppe, lorsque le au moins un repère est dans une position prévue pour lui à l'intérieur de l'enveloppe.

7. Utilisation selon l'une des revendications précédente, dans laquelle au moins un repère est utilisé à des fins de navigation médicale.

8. Utilisation selon l'une des revendications précédentes, dans laquelle les repères entourés par l'enveloppe de repère sont utilisés pour un système de poursuite et des caméras pour la détection d'un rayonnement optique des repères enveloppés.

9. Utilisation selon la revendication 8, dans laquelle le système de poursuite délivre un signal d'avertissement et/ou au moins un des repères enveloppés ne peut plus être détecté lorsque le capteur constate de l'humidité ou un liquide ou vient en contact avec ceux-ci.

10. Utilisation selon l'une des revendications précédents, dans laquelle les rayons optiques partent du au moins un repère par suite d'une réflexion ou d'un rayonnement.
